# EUROPÄISCHE PATENTSCHRIFT

(11) **EP 0 559 064 B1**
(45) Veröffentlichungstag und Bekanntmachung des Hinweises auf die Patenterteilung: **23.05.2001**
(21) Anmeldenummer: 93102848.4
(22) Anmeldetag: 24.02.1993
(51) Int. Cl.: C12N 9/16, C12N 9/18, C07J 9/00, C07K 1/00

(54) **Komplexe enthaltend Glykosyl-Phosphatidylinositol-Proteine und Cholansäurederivate, Verfahren zu ihrer Herstellung und ihre Verwendung**
Complexes containing glycosyl-phosphatidylinositol-proteins and cholanic acid-derivatives, process for their preparation and their use
Complexes contenant des glycosyl-phosphaditylinositol-protéines et des dérivés de l'acide cholanique, procédé pour leur préparation et leur utilisation

(30) Priorität: 29.02.1992 DE 4206395
(43) Veröffentlichungstag der Anmeldung: 08.09.1993
(73) Patentinhaber: Aventis Research & Technologies GmbH & Co. KG, 65926 Frankfurt am Main (DE)
(72) Erfinder: Müllner, Stefan, Dr., W-6203 Hochheim (DE); Müller, Günter, Dr., W-6231 Sulzbach/Taunus (DE)
(74) Vertreter: Ackermann, Joachim, Dr.

(56) Entgegenhaltungen:
- DE-A- 3 623 747
- JOURNAL OF BIOLOGICAL CHEMISTRY. Bd. 262, Nr. 15 , 25. Mai 1987 , BALTIMORE, MD US Seiten 7087 - 7091 HEIDEMAN W. U.A. 'Adenyl Cyclase in Yeast'
- PROCEEDINGS OF THE NATIONAL ACADEMY OF SCIENCES OF USA Bd. 85 , Februar 1988 , WASHINGTON US Seiten 980 - 984 LOW M.G. U.A.. 'A phospholipase D specific for the phosphatidinyinositol anchor of cell-surface proteins is abundant in plasma'
- JOURNAL OF BIOLOGICAL CHEMISTRY. Bd. 265, Nr. 29 , 15. Oktober 1990 , BALTIMORE, MD US Seiten 17738 - 17745 HUANG K. U.A. 'Purification and Characterization of Glycosyl-phosphatidylinositol specific Phospholipase D'

## Beschreibung

Integrale Membranproteine, welche mit einem oder mehreren Abschnitten der Polypeptidkette in die Lipiddoppelschicht eingelagert sind, sind meist wasserunlöslich und nur mit Lösungsvermittlern, sogenannten Detergentien, unter Wahrung ihrer Struktur in eine wasserlösliche Form zu bringen.

Unter der Vielzahl von möglichen Detergentien sind auch Cholansäurederivate bekannt (DE 36 23 747), mit denen einige Membranproteine in eine wasserlösliche Form gebracht werden können. Es ist ferner bekannt, daß diese Cholansäurederivate bevorzugt Proteine aus Plasmamembranen in eine wasserlösliche Form bringen (solubilisieren). Eine Selektivität für bestimmte Membranproteine ist bisher nicht bekannt.

Seit einiger Zeit ist eine Klasse von Membranproteinen bekannt, welche kovalent über einen Glykosyl-Phosphatidylinositol-Anker an die Plasmamembranen gebunden sind (G. A. M. Cross (1987) Cell 48, S. 179 - 181; M. G. Low (1987) Biochem. J. 244, 1 - 13). Die Solubilisierung dieser Membranproteine, z. B. alkalische Phosphatasen in funktioneller Form, wird bislang nur durch Behandlung mit Protease wie Trypsin oder Papain erreicht oder nach Freisetzung durch bakterielle Phospholipase-C (I. F. Thompson et al. (1987) Biochem. Biophys. Res. Commun. 145, 118- 125). Nun erhält man hierbei nur den hydrophilen Teil der Membranproteine. Für strukturelle und funktionelle Untersuchungen sollte aber die amphipatische Form mit intaktem Membrananker vorliegen. Membranproteine mit Glykosyl-Phosphatidylinositol-Anker (GPI-Proteine) können mit herkömmlichen Detergentien nur gemeinsam mit anderen Membranproteinen solubilisiert werden. Hierfür wird oft auch die Extraktion mit n-Butanol eingesetzt (A. S. Malik and M. G. Low (1986) Biochem. J. 240, S. 519 - 527). Bei dieser Form der Extraktion kann jedoch häufig die ursprüngliche räumliche Struktur dieser Membranproteine verloren gehen. Dies zeigt sich beispielsweise in einer verminderten Zugänglichkeit des GPI-Membranankers für die Spaltung durch GPI-spezifische Phospholipasen.

Er wurde nun gefunden, daß GPI-Proteine im wesentlichen ihre ursprüngliche räumliche Struktur sowie ihre Aktivität und Funktionalität behalten, wenn sie aus der Zellmembran schonend mit Hilfe der Cholansäurederivate in eine wasserlösliche Form gebracht werden. Diese so erhaltenen wasserlöslichen Komplexe lassen sich mit Phospholipasen selektiv und in hoher Ausbeute in einen Phosphoinositol-Glykan-Proteinanteil und Diacylglyzerin spalten. Ferner lassen sich die Glykosyl-Phosphatidyinositol-Proteine (GPI-Proteine) mit den Cholansäurederivaten selektiv von anderen Membranproteinen abtrennen.

Die Erfindung betrifft daher wasserlösliche Komplexe, enthaltend mindestens ein Glykosyl-Phosphatidylinositol-Protein und mindestens ein Cholansäurederivat.

Die erfindungsgemäßen wasserlöslichen Komplexe haben die Eigenschaft, daß sie in wässriger Lösung bei neutralem pH-Wert erst dann sedimentierbare Aggregate bilden, wenn sie in einer Zentrifuge 30 Minuten mit mehr als der 100 000fachen Erdbeschleunigung (g) rotiert werden.

Bei den Cholansäurederivaten handelt es sich um Verbindungen der Formel I 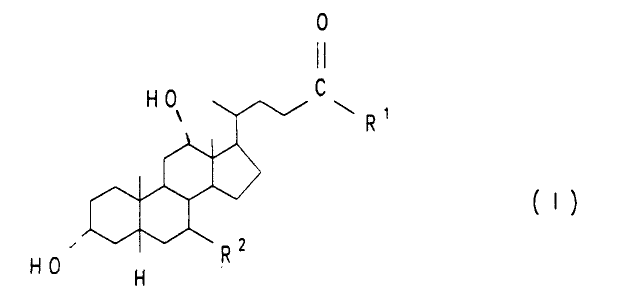 und/oder physiologisch verträgliche Salze der Verbindung der Formel I; wobei die Symbole R¹ und R² die nachstehende Bedeutung haben:
- R¹: bedeutet:
a) -NH-CH₂-CH₂-SO₃ oder
b) -NH-CH₂-COOH,
- R2: bedeutet:
a) -N-R³ oder
   R⁴
b) -O-R⁵,
   dabei sind R³, R⁴ oder R⁵ gleich oder verschieden und haben unabhängig voneinander die folgende Bedeutung:
   1) Wasserstoffatom,
   2) (C₁-C₅)-Alkyl, geradkettig oder verzweigt,
   3) (C₃-C₆)-Cycloalkyl,
   4) Acetyl,
   5) Acetyl, ein- oder mehrfach substituiert durch
      5.1 Halogen wie Fluor, Chlor oder Brom,
   6) Halogen wie Fluor, Chlor oder Brom,
   7) Succinyl,
   8) Benzoyl, ein- oder mehrfach substituiert durch
      8.1 Wasserstoffatom,
      8.2 -NO₂,
      8.3 -N₃,
      8.4 -NCS,
      8.5 -NH₂,
      8.6 -N(R⁷)₂, dabei hat R⁷ die Bedeutung von b 1) bis b 7) oder
      8.7 Halogen wie Fluor, Chlor oder Brom oder
   9) Benzyl, ein oder mehr substituiert durch die in b 8.1 bis b 8.7 genannten Reste.

Bevorzugt sind Verbindungen der Formel I;
wobei
- R¹: bedeutet -NH-CH₂-CH₂-SO₃ und
R2 bedeutet -NHR⁴, dabei hat R⁴ die folgende Bedeutung:
a) (C₁-C₅)-Alkyl,
b) Benzoyl, ein- oder mehrfach substituiert durch
   1) Wasserstoffatom,
   2) NO₂,
   3) NH₂ oder
   4) Halogen wie Fluor, Chlor oder Brom oder
c) Benzyl, ein- oder mehrfach substituiert durch die in b 1) bis b 4) genannten Reste.

Insbesondere bevorzugt sind Verbindungen der Formel I;
wobei
- R¹: bedeutet -NH-CH₂-CH₂-SO₃ und
- R²: bedeutet -NH-R⁴, dabei hat R⁴ die folgende Bedeutung:
a) Benzoyl oder
b) Benzoyl substituiert durch NH₂.

Ganz besonders bevorzugt ist 4'-Amino-7-benzamido-3α,12α-5β-taurocholsäure als Cholansäurederivat.

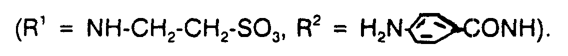

Geeignete physiologisch verträgliche Salze der erfindungsgemäßen Cholansäurederivate sind beispielsweise Alkali-, Erdalkali- oder Ammoniumsalze, sowie Salze mit physiologisch verträglichen, organischen Ammonium- oder Triethylaminbasen. Unter dem Begriff Glykosyl-Phosphatidylinositol-Proteine (GPI-Proteine) werden Verbindungen verstanden, die über ihren Glykosyl-Phosphatidylinositol-Teil in einer Zellmembran von Mikroorganismen, Pflanzenzellen, Pilzzellen oder Tierzellen verankert sind und einen Proteinanteil besitzen. Beispiele für GPI-Proteine sind 5'-Nukleotidase, alkalische Phosphatase und Acetylcholinesterase.

Die obengenannten Cholsäurederivate lassen sich nach literaturbekannten Verfahren herstellen (DE 36 23 747).

Zur Herstellung der 7-Amino-Cholsäure wird beispielsweise Cholsäure nach Fieser und Rajagopolan (IACS 71, 3935) in 7-Stellung oxidiert, nach Redel et al. (Bull. Soc. Chin. Fr. 1949, S. 877) zum Oxim umgewandelt und nach Tserng et al. (J. Lipid Res. 18, 404, 1977) mit Taurin konjugiert sowie anschließend unter Druck am Platinkontakt zum Amin hydriert. Das Amin wird anschließend mit Nitroarylalkohol oder einer aromatischen Nitrocarbonsäure, bevorzugt 4-Nitrobenzoesäure, unter Zusatz von N-Ethoxycarbonyl-2-ethoxy-1,2-dihydrochinolin (EEDQ) in Dimethylformamind (DMF) gekuppelt und das 4-Nitrobenzamid wird in essigsaurer methanolischer Lösung mit PtO₂xH₂O als Katalysator zur Aminoverbindung, bevorzugt zum 4-Aminobenzoesäurederivat,hydriert.

Die Synthese der 3- und/oder 7-Hydroxy-Taurocholsäureester erfolgt analog durch Umsetzen der entsprechenden Hydroxyverbindung mit einem Nitroarylalkohol oder einer aromatischen Nitrocarbonsäure, bevorzugt 4-Nitrobenzoesäure,unter Zusatz von EEDQ in DMF. Anschließend wird, wie beschrieben, am PtO₂-Kontakt hydriert.

Die Herstellung der erfindungsgemäßen wasserlöslichen Komplexe erfolgt beispielsweise dadurch, daß man
a) Membranen, die GPI-Proteine enthalten, isoliert,
b) die Membranen mit mindestens einem Cholansäurederivat inkubiert und
c) wasserlösliche Komplexe, enthaltend mindestens ein GPI-Protein und mindestens ein Cholansäurederivat, isoliert.

GPI-Proteine können aus zahlreichen Organismen gewonnen werden, beispielsweise aus Ratten, Schweinen oder Rindern. Geeignet sind beispielsweise Plasmamembranen aus Rattenadipozyten zur Gewinnung von 5'-Nukleotidase oder Membranen aus Rindererythrozyten zur Gewinnung von Acetylcholinesterase.

Beim Verfahrensschritt a) geht man am besten wie folgt vor: Membranen, die GPI-Proteine enthalten, werden isoliert, indem beispielsweise Adipozyten aus Ratten-Nebenhodenfettgewebe durch Kollagenaseverdau (J. Gliemann, Diabetologia, 1967, 3, S. 382 - 388) vereinzelt werden. Die isolierten Zellen werden mit einem Homogenisator zerkleinert, und die Plasmamembranen werden durch differentielle Zentrifugation angereichert und anschließend durch Saccharosegradiertenzentrifugation (15 bis 40 % Saccharose) gereinigt (D. W. McKeel und L. Jarret, J. Cell. Biol., 1970, 44, S. 417- 432).

Beim Verfahrensschritt b) geht man am besten so vor, daß man die isolierten Membranen mit einem Cholansäurederivat beispielsweise 4'-Amino-7β-bezamido-3α,12α,5β-taurocholsäure (BATC) inkubiert. Die Konzentration der Cholansäurederivate beträgt etwa 0,01 bis 0,5 %, bevorzugt 0,02 bis 0,2 %, besonders bevorzugt 0,05 bis 0,1 %. Die Temperatur beträgt von 0° C bis 40° C, bevorzugt 0° C bis 15° C. Die Inkubationszeit beträgt von 10 min bis 2 Stunden, bevorzugt 15 min bis 1 Stunde, ganz besonders bevorzugt 20 min bis 40 min.

Beim Verfahrensschritt c) geht man am besten so vor, daß man die in Schritt b) erhaltene Reaktionsmischung mit 35 000 bis 150 000 *g, bevorzugt 50 000 bis 100 000 *g, zentrifugiert. Es kann ein- oder mehrmal zentrifugiert werden; die Zentrifugalbeschleunigung kann während eines Zentrifugationsschrittes gleich sein oder variieren. Die Zentrifugalbeschleunigung kann bei mehreren Zentrifugationsvorgängen gleich oder verschieden sein. Die Zentrifugationszeit kann in einem weiten Bereich schwanken, als günstig haben sich Zentrifugationszeiten von 15 min bis zu 3 Stunden erwiesen, bevorzugt 30 min bis 1 Stunde. Die erfindungsgemäßen wasserlöslichen Komplexe befinden sich im Überstand nach dem Zentrifugationsschritt. Sie können dann als solche in Lösung verwendet werden oder gegebenenfalls eingefroren oder weiter gereinigt werden. Entsprechende Methoden sind literaturbekannt, beispielsweise:
Brodbeck, U.; Gentinetta, R.; Ott, P. (1981) in "Membrane Proteins" (Azzi A., Brodbeck U., Zahler P. eds.) Springer-Verlag, Berlin, 85 - 96.
Stieger, S.; Brodbeck, U. (1985), J. Neurochem. 44, 48 - 56;
Butikofer, P.; Kuypers, F. A.; Shackleton, C.; Brodbeck, U.; Stieger, S. (1990), J. Biol. Chem. 265, 18983 - 18987.
Ott, P.; Jenny, B.; Brodbeck, U. (1975), Eur. J. Biochem 57, 469 - 480.

Die so erhaltenen wasserlöslichen Komplexe eignen sich beispielsweise in Enzymtests, zur qualitiven oder quantitativen Bestimmung von Substraten in chemischen Synthesen oder zur Stabilisierung von Testsystemen in denen jeweils GPI-Proteine anwesend sind, und zur Reinigung von GPI-Proteinen.

Die Erfindung betrifft ferner ein Verfahren zur selektiven Gewinnung der erfindungsgemäßen wasserlöslichen Komplexe. Die obengenannte Gewinnung der wasserlöslichen Komplexe zeichnet sich durch eine hohe Selektivität der Cholansäurederivate für die GPI-Proteine aus. Andere Detergentien wie Octylglukosid oder Triton X-100 weisen eine signifikant schlechtere Selektivität für die GPI-Proteine auf.

Die Prozentangaben in den folgenden Beispielen beziehen sich jeweils auf Volumenprozente, falls nicht anders angegeben.

### Beispiel 1

### Wasserlöslicher 5'-Nukleotidase-BATC-Komplex

a) Adipozyten werden durch Kollagenaseverdau aus Ratten-Nebenhodenfettgewebe isoliert (nach J. Gliemann (1967) Diabetologia 3, S. 382 - 388), gewaschen und in einem Glas-Homogenisator (B. Braun, Melsungen AG, Apparatebau) homogenisiert. Nach differentieller Zentrifugation des Homogenats (5 Minuten bei 1000*g und nachfolgend 20 min bei 16 000 ^{*}g) wird das resultierende Plasmamembranpellet in einem wässrigen Puffer 25 mM Tris/HCl (pH 7,4, 250 mM Saccharose, 1 mM EDTA) suspendiert und durch Saccharosegradientenzentrifugation (linearer Gradient von 15 - 40 % Saccharose in 25 mM Tris/HCl) gereinigt (D. W. McKeel and L. Jarrett (1970) J. Cell. Biol. 44, S. 417 - 432).
b) Die Plasmamembranen aus a) werden mit 0,1 % 4'-Amino-7β-benzamido-3α,12α,5β-taurocholsäure (BATC) inkubiert. Die Reaktionszeit beträgt 30 min bei 4° C.
c) Der Inkubationsansatz aus b) wird 30 min bei 150 000 *g zentrifugiert, die sedimentierten Plasmamembranen werden verworfen und der Überstand wird anschließend noch zweimal 30 min bei 100 000 ^{*}g zentrifugiert und die sedimentierten Membranen werden jeweils verworfen. Der Überstand enthält den wasserlöslichen 5'-Nukleotidase-BATC-Komplex.

Der Nachweis der 5'-Nukleotidaseaktivität erfolgt durch Hydrolyse von radioaktivem 5'-AMP. Nach Präzipitation des nicht hydrolisierten AMP durch 0,3 N Ba(OH)₂ wird die Radioaktivität des entstandenen Adenosins im Überstand gemessen (nach E. M. Bailyes et al. (1982) Biochem. J. 203, S. 245 - 251).

### Beispiel 2

### Wasserlöslicher Alkalische Phosphatase-BATC-Komplex

Die Herstellung erfolgt wie in Beispiel 1 a) bis 1 c) beschrieben.

Der Nachweis der alkalischen Phosphataseaktivität erfolgt spektroskopisch durch Hydrolyse von p-Nitrophenylphosphat (Y. Ikehara et al. (1977) Biochem. Biophys. Acta 470, S. 202 - 211).

### Beispiel 3

### Wasserlöslicher Acetylcholinesterase-BATC-Komplex

Es werden Rindererythrozten (Sigma Chemie GmbH, Deisenhofen, Germany) wie in Beispiel 1 b) und 1 c) beschrieben inkubiert. Dazu werden 1 mg getrocknete Rindererythrozyten in 1 ml Puffer (Tris/HCl, 25 mM, pH 7,4) mit 0,1 % BATC (bezogen auf das Volumen) 30 min bei 4° C inkubiert und anschließend wie in Beipiel 1 c) zentrifugiert.

Der Nachweis der Acetylcholinesterase (T. L. Rosenberry and D. M. Scoggin (1984) J. Biol. Chem. 259, S. 5643 - 5652) erfolgt über die Hydrolyse von radioaktivem Acetylcholin. Die Radioaktivität der freigesetzten radioaktiven Essigsäure wird nach Abtrennen des nicht hydrolysierten radioaktiven Acetylcholins durch Phasentrennung bei saurem pH-Wert gemessen.

### Beispiel 4

### Selektive Anreicherung GPI-Proteinen aus Zellmembranen

Die selektive Anreicherung von GPI-verankerten Membranproteinen durch BATC im Vergleich zu herkömmlichen in der Membranbiochemie eingesetzten Detergentien erfolgt folgendermaßen:

Rindererythrozytenmembranen und Rattenfettzellplasmamembranen (hergestellt wie in Beispiel 1 a) oder 3 a)) werden in Gegenwart von BATC (0,1 %), Octylglukosid (0,5 %), und Triton TX-100 (0,5 %) inkubiert. Nach Zentrifugation (30 min 100 000 x g) werden die spezifischen enzymatischen Aktivitäten der 5'-Nukleotidase, alkalischen Phosphatase und Acetylcholinesterase, im Überstand bestimmt und jeweils als Anreicherungsfaktor gegenüber der gesamten spezifischen Aktivität im detergenzsolubilisierten Inkubationsansatz vor der Zentrifugation berechnet. Tabelle 1 zeigt die Ergebnisse

| | BATC 0,1 % | Octylglycoside 0,5 % Anreicherungsfaktor | Triton X-100 0,5 % |
|---|---|---|---|
| 5'-Nukleotidase | 4,4 | 1,5 | 1,0 |
| Alkalische Phosphatase | 3,5 | 1,2 | 0,8 |
| Acetylcholinesterase | 4,8 | 1,6 | 0,9 |

### Beispiel 5

### Lipolytische Spaltung vom GPI-Protein 5'-Nukleotidase

Zur Demonstration der effizienten lipolytischen Spaltung von GPI-Proteinen durch bakterielle Pl(Phosphatidylinositol)-spezifische Phospholipasen in Gegenwart von BATC werden Rattenfettzellplasmamembranen (Beispiel 1) mit PI-PLC (Bacillus cerus, Sigma Chemie GmbH) in Gegenwart von 0,1 % BATC, 0,5 % Deoxycholat oder 0,1 % Nonidet P-40 inkubiert.

Die Spaltung des Glykosyl-Phosphatidylinositol-Membranankers der 5'-Nukleotidase durch (G)PI-PLC(D) aus Bacillus cereus wird durch das Verteilungsverhalten der Proteine (identifiziert durch ihre enzymatische Aktivität) zwischen einer Detergenz-(TX-114) und einer wässrigen Phase verfolgt. Die amphiphile Membrananker-haltige Form reichert sich in der Detergenzphase an, die hydrophile Membrananker-freie Form wird in der wässrigen Phase konzentriert.

Die Phasentrennung mit TX-114 erfolgt nach C. Bordier (1981) J. Biol. Chem. 256, S. 1604- 1607; J. G. Pryde and J. H. Phillips (1986) Biochem. J. 233, S. 525 - 533; B. R. Ganong and J. P. Delmore (1991) Anal. Biochem. 193, S. 35- 37 mit folgenden Modifikationen: 50 *µ*l Inkubationsansatz werden mit 1 ml eiskaltem TX-114 (2 %), 144 mM NACI versetzt. Die Phasentrennung wird durch Aufwärmen auf 37° C und Zentrifugation (2 min bei 10 000 * g) herbeigeführt. Die Detergenzphase wird zweimal reextrahiert. Die wässrigen Phasen wurden vereinigt. Tabelle 2 zeigt die Ergebnisse:

| Inkubationszeit | Detergenz | | |
|---|---|---|---|
| (min) | BATC 0,1 % | Nonidet P-40 0,1 % | Deoxycholat 0,5 % |
| 0 | 27 | 33 | 6 |
| 5 | 55 | 48 | 8 |
| 30 | 112 | 88 | 11 |
| 60 | 256 | 211 | 19 |
| 90 | 578 | 485 | 45 |
| 120 | 732 | 654 | 109 |
| 240 | 1842 | 1489 | 144 |
| 480 | 2231 | 1651 | 162 |

Die lipolytische Spaltung der GPI-Membrananker mit (G)PI-PLC's in Gegenwart von BATC (0,1 %) erfolgt im Vergleich mit Nonidet P-40 oder Deoxycholat schneller und mit deutlich höherer Effizienz.

## Patentansprüche

1. Wasserlöslicher Komplex, enthaltend mindestens ein Glykosyl-Phosphatidylinositol-Protein und mindestens ein Cholansäurederivat der Formel I 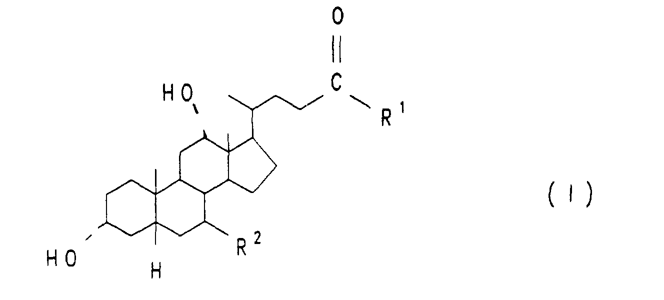 und/oder physiologisch verträgliche Salze der Verbindung der Formel I; wobei die Symbole R¹ und R² die nachstehenden Bedeutungen haben:
R¹ bedeutet:
a) -NH-CH₂-CH₂-SO₃ oder
b) -NH-CH₂-COOH,
R² bedeutet
a) 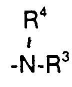 oder
b) -O-R⁵,
dabei sind R³, R⁴ oder R⁵ gleich oder verschieden und haben unabhängig voneinander die folgende Bedeutung:
1) Wasserstoffatom,
2) (C₁-C₅)-Alkyl, geradkettig oder verzweigt,
3) (C₃-C₆)-Cycloalkyl,
4) Acetyl,
5) Acetyl, ein- oder mehrfach substituiert durch
5.1 Halogen wie Fluor, Chlor oder Brom,
6) Halogen wie Fluor, Chlor oder Brom,
7) Succinyl,
8) Benzoyl, ein- oder mehrfach substituiert durch
8.1 Wasserstoffatom,
8.2 -NO₂,
8.3 -N₃,
8.4 -NCS,
8.5 -NH₂,
8.6 -N(R⁷)₂, dabei hat R⁷ die Bedeutung von b 1) bis b 4) oder
8.7 Halogen wie Fluor, Chlor oder Brom oder
9) Benzyl, ein oder mehr substituiert durch die in b 8.1 bis b 8.7 genannten Reste.

2. Wasserlöslicher Komplex nach Anspruch 1, wobei
R¹ bedeutet -NH-CH₂-CH₂-SO₃ und
R² bedeutet -NHR⁴, dabei hat R⁴ die folgende Bedeutung:
a) (C₁-C₅)-Alkyl,
b) Benzoyl, ein- oder mehrfach substituiert durch
1) Wasserstoffatom,
2) NO₂,
3) NH₂ oder
4) Halogen wie Fluor, Chlor oder Brom oder
c) Benzyl, ein- oder mehrfach substituiert durch die in b 1) bis b 4) genannten Reste.

3. Wasserlöslicher Komplex nach Anspruch 1 oder 2, wobei
R¹ bedeutet -NH-CH₂-CH₂-SO₃ und
R² bedeutet -NHR⁴, dabei hat R⁴ die folgende Bedeutung:
a) Benzoyl oder
b) Benzoyl substituiert durch NH₂.

4. Wasserlöslicher Komplex nach einem oder mehreren der Ansprüche 1 bis 3, wobei als Cholansäurederivat 4'-Amino-7-benzamido-3α,12α,-5β-taurocholsäure enthalten ist.

5. Wasserlöslicher Komplex nach einem oder mehreren der Ansprüche 1 bis 4, wobei als Glykosyl-Phosphatidylinositol-Protein 5'-Nukleotidase, alkalische Phosphatase oder Acetylcholinesterase enthalten ist.

6. Verfahren zur Herstellung eines wasserlöslichen Komplexes nach einem oder mehreren der Ansprüche 1 bis 5, dadurch gekennzeichnet, daß man
a) Membranen, die Glykosyl-Phosphatidylinositol-Proteine enthalten, isoliert,
b) die Membranen mit mindestens einem Cholansäurederivat inkubiert und
c) einen wasserlöslichen Komplex, enthaltend mindestens ein Glykosyl-Phosphatidylinositol-Protein und mindestens ein Cholansäurederivat, isoliert.

7. Verwendung von mindestens einem wasserlöslichen Komplex nach Anspruch 1 bis 5 zum Einsatz in Enzymtests oder chemischen Synthesen oder bei der Reinigung von Glykosyl-Phosphatidyl-inositol-Proteinen.

## Claims

1. A water-soluble complex containing at least one glycosyl-phosphatidylinositol protein and at least one cholanic acid derivative of the formula I 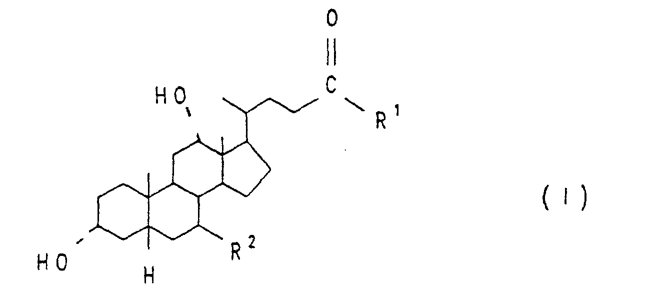 and/or physiologically tolerated salts of the compound of the formula I; in which the symbols R¹ and R² have the following meaning:
R¹ is:
a) -NH-CH₂-CH₂-SO₃ or
b) -NB-CH₂-COOH,
R² is:
a) 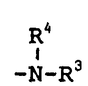 or
b) -O-R⁵,
where R³, R⁴ or R⁵ are identical or different and have, independently of each other, the following meaning:
1) hydrogen atom,
2) (C₁-C₅)-alkyl, straight-chain or branched,
3) (C₃-C₆)-cycloalkyl,
4) acetyl,
5) acetyl, monosubstituted or polysubstituted by 5.1 halogen such as fluorine, chlorine or bromine,
6) halogen such as fluorine, chlorine or bromine,
7) succinyl,
8) benzoyl, monosubstituted or polysubstituted by
8.1 hydrogen atom
8.2 -NO₂,
8.3 -N₃,
8.4 -NCS,
8.5 -NH₂,
8.6 -N(R⁷)₂, where R⁷ has the meaning of b 1) to b 4) or
8.7 halogen such as fluorine, chlorine or bromine or
9) benzyl, monosubstituted or polysubstituted by the radicals named in b 8.1 to b 8.7.

2. A water-soluble complex as claimed in claim 1, wherein
R¹ is -NH-CH₂-CH₂-SO₃ and
R² is -NHR⁴, where R⁴ has the following meaning:
a) (C₁-C₅)-alkyl,
b) benzoyl, monosubstituted or polysubstituted by
1) hydrogen atom,
2) NO₂,
3) NH₂ or
4) halogen such as fluorine, chlorine or bromine or
c) benzyl, monosubstituted or polysubstituted by the radicals named in b 1) to b 4).

3. A water-soluble complex as claimed in claim 1 or 2, wherein
R¹ is -NH-CH₂-CH₂-SO₃ and
R² is -NH-R⁴, where R⁴ has the following meaning:
a) benzoyl or
b) benzoyl substituted by NH₂.

4. A water-soluble complex as claimed in one or more of claims 1 to 3, which contains 4'-amino-7-benzamido-3α,12α,-5β-taurocholic acid as the cholanic acid derivative.

5. A water-soluble complex as claimed in one or more of claims 1 to 4, which contains 5'-nucleotidase, alkaline phosphatase or acetylcholinesterase as the glycosyl-phosphatidylinositol protein.

6. A process for preparing a water-soluble complex as claimed in one or more of claims 1 to 5, which comprises
a) isolating membranes which contain glycosyl-phosphatidylinositol proteins,
b) incubating the membranes with at least one cholanic acid derivative and
c) isolating a water-soluble complex containing at least one glycosyl-phosphatidylinositol protein and at least one cholanic acid derivative.

7. The use of at least one water-soluble complex as claimed in claims 1 to 5 for employment in enzyme tests or chemical syntheses or in the purification of glycosyl-phosphatidylinositol proteins.

## Revendications

1. Complexe hydrosoluble, contenant au moins une protéine glycosyl-phosphatidyl-inositol et au moins un dérivé de l'acide cholanique de formule I 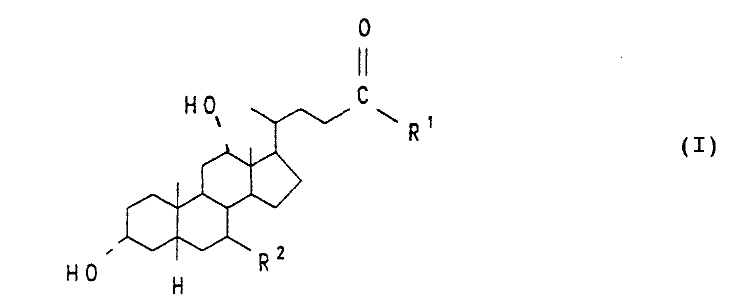 et/ou des sels tolérés sur le plan physiologique du composé de formule I ;
les symboles R¹ et R² possèdent les significations suivantes :
R¹ représente
a) -NH-CH₂-CH₂-SO₃ ou
b) -NH-CH₂-COOH,
R² représente
a) 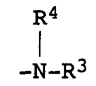 ou
b) -O-R⁵,
R³, R⁴ ou R⁵ étant identiques ou différents et possèdent, indépendamment les uns des autres, les significations suivantes :
1) un atome d'hydrogène,
2) un groupe alkyle en C₁-C₅, à chaîne droite ou ramifiée
3) un groupe cycloalkyle en C₃-C₆,
4) un groupe acétyle,
5) un groupe acétyle substitué une ou plusieurs fois par 5.1 un atome d'halogène comme le fluor, le chlore ou de brome,
6) un atome d'halogène comme le fluor, le chlore ou de brome,
7) un groupe succinyle,
8) un groupe benzoyle substitué une ou plusieurs fois par
8.1 un atome d'hydrogène,
8.2 -NO₂,
8.3 -N₃,
8.4 -NCS,
8.5 -NH₂,
8.6 -N(R⁷)₂, R⁷ ayant les significations données de b 1) à b 4) ou
8.7 un atome d'halogène comme le fluor, le chlore ou de brome, ou
9) un groupe benzyle substitué une ou plusieurs fois par les restes cités aux points b 8.1 à b 8.7.

2. Complexe hydrosoluble selon la revendication 1, où
R¹ représente -NH-CH₂-CH₂-SO₃ et
R² représente -NHR⁴, R⁴ possédant les significations suivantes :
a) un groupe alkyle en C₁-C₅,
b) un groupe benzoyle substitué une ou plusieurs fois par
1) un atome d'hydrogène,
2) -NO₂,
3) -NH₂ ou
4) un atome d'halogène comme le fluor, le chlore ou de brome, ou
c) un groupe benzyle substitué une ou plusieurs fois par les restes cités aux points b 1) à b 4).

3. Complexe hydrosoluble selon la revendication 1 ou 2, où
R¹ représente -NH-CH₂-CH₂-SO₃ et
R² représente -NHR⁴, R⁴ possédant les significations suivantes :
a) un groupe benzoyle ou
b) un groupe benzoyle substitué une ou plusieurs fois par NH₂ .

4. Complexe hydrosoluble selon une ou plusieurs revendications 1 à 3, contenant l'acide 4'-amino-7-benzamido-3α,12α,5β-taurocholique en tant que le dérivé de l'acide cholanique.

5. Complexe hydrosoluble selon une ou plusieurs revendications 1 à 4, contenant la 5'-nucléotidase, la phosphatase alcaline ou l'acétylcholine estérase en tant que protéine glycosyl-phosphatidyl-inositol.

6. Procédé pour la préparation d'un complexe hydrosoluble selon une ou plusieurs des revendications 1 à 5, caractérisé en ce qu'on
a) isole les membranes contenant des protéines GPI,
b) incube les membranes avec au moins un dérivé de l'acide cholanique et
c) isole un complexe hydrosoluble, contenant au moins une protéine GPI et au moins un dérivé de l'acide cholanique.

7. Emploi d'au moins un complexe hydrosoluble selon la revendication 1 à 5 pour l'utilisation dans des tests enzymatiques ou synthèses chimiques ou dans la purification de protéines glycosyl-phosphatidyl-inositol.
